# EUROPEAN PATENT APPLICATION

(11) **EP 3 576 094 A1**
(43) Date of publication of application: **04.12.2019**
(21) Application number: 18174969.8
(22) Date of filing: 29.05.2018
(51) Int. Cl.: G16H 20/30

(54) **METHOD FOR MEASURING OVERREACHING**

(71) Applicant: ProteiQ Biosciences GmbH, 10967 Berlin (DE)
(72) Inventor: Pugachev, Artyom, 10967 Berlin (DE); Groen, Arnoud Johan, 10967 Berlin (DE)
(74) Representative: Wulferink, Marty Bernardus Franciscus

(57) **Abstract**

The invention relates to a method for measuring stress in general, in particular physical stress caused by, e.g., sports, training, or (physical) trauma, and/or psychological stress caused by, e.g., work or (psychological) trauma, preferably with the aim to assist with the decision making to change a specific training regimen or life style. In particular, the invention relates to measuring expression of specific proteins in bodily samples. More particular, the invention relates to measuring proteins that are related to the status of the immune system. The invention further relates to means, in particular kits of parts, that enable measuring stress-related proteins in bodily samples.

## Description

### Field of the Invention

The invention relates to a method for measuring stress in general, in particular physical stress caused by, e.g., sports, training, or (physical) trauma, and/or psychological stress caused by, e.g., work or (psychological) trauma, preferably with the aim to assist with the decision making to change a specific training regimen or life style.

### Background

Currently there is no method to reliably identify "overreaching" or "overtraining". Two out of ten professional athletes overreach, as part of normal training. If managed correctly, overreaching may increase the performance. However, if pushed too far the body cannot recover. Typical symptoms are comparable to burn-out in non-athletes, leading to reduction in performance and motivation. Once it has happened it takes from 2 weeks to several years to recover. It is a risk to be managed. Target markets are individual and team endurance sports (i.e. swimming, cycling, rowing and running).

Successful training leading to enhanced performance involves cycles of overload and adequate recovery. A primary goal during training is to avoid the combination of excessive overload and inadequate recovery leading to "overreaching," defined as a short-term decrement in performance with or without related physiological and psychological symptoms in which restoration of performance takes several days to several weeks (Meeusen, et al.; Med. Sci. Sports Exerc. 2013, 45, 186-205). "Functional" overreaching occurs when athletes deliberately use a short-term period (e.g. training camp) to increase the training load resulting in short-term performance decrements without serious, long-lasting psychological or other negative symptoms (Aubry et al.; Med. Sci. Sports Exerc. 2014, 46, 1769-1777).

"Functional overreaching" (FOR or short-term overreaching) will eventually lead to an improvement in performance after recovery. Non-functional overreaching (NFOR or extreme overreaching) occurs when athletes train beyond their ability to recover with concomitant performance decrements and psychological disturbances that include decreased vigor and energy, increased fatigue, and loss of desire to train (Meeusen, et al.; Med. Sci. Sports Exerc. 2013, 45, 186-205). NFOR can result in a prolonged recovery time with sleep disturbance, elevated resting heart rate, illness, and psychological stress. A hallmark feature of FOR and NFOR is the inability to sustain intense exercise for a prolonged period of time. NFOR can easily progress to the overtraining syndrome (OTS), and athletes with OTS may take months or possibly years to completely recover.

The inventors have been in personal contact with a hobby athlete team (Valle) from a small Italian town that has always been a top finisher in the local yearly competition. This year, the new president decided that they should train more and harder and starting one month earlier. Shortly before the competition, half of the team got sick and the team finished last this year. This is a clear case that training more is not always better and although most people are aware of this, they don't tend to admit to it. This is a clear example where our panel could have warned against this too intense training load. This will advise them to moderate their training schedule and to take the time to recover.

Michael Wolfe is an American ultra-distance athlete who, in hind sight, had suffered from overtraining. After finishing a race at a disappointing 13^{th} place he said: "It wasn't like my stomach was bad or I couldn't eat. It was like my body was just shut down. Like, nope, you can't run that hard. I'm not going to allow it."

Like Michael there are many other recent examples of athletes that have simply trained too hard since they believed that training harder would make them better including Anna Frost, who won the women's division of the North Face Endurance Championship in 2011; Anton Krupicka, two-time winner of the Leadville 100; Geoff Roes, who set a new record at the 2010 Western States 100; and Kyle Skaggs, who demolished the Hardrock 100 record in 2008. Each of them reached the absolute top of their sport only to mysteriously struggle to repeat their results.

Perhaps the most famous example of severe non-functional overreaching/ overtraining is Alberto Salazar, now an athletics coach to many elite athletes. During his last years of his career he was plagued by respiratory infections and depression. When he retired in 1998, he was barely able to complete runs longer than 30 minutes.

David Nieman, Professor at the Human Performance Laboratory at the Appalachian State University says: "OTS is one of the scariest things I've ever seen in my 30 plus years of working with athletes," and "To watch someone go from that degree of proficiency to a shell of their former self is unbelievably painful and frustrating."

Every Olympics there are athletes that mysteriously underperform and who have trained extremely hard to get to the finals. Many athletes often just get ill right before an important stressful race, even if they are not elite athletes. The existence of this threat to an athlete's health will never be carefully treated if there is no objective way of measuring it.

Up to the present invention, few biochemical tests exist that claim to be able to measure FOR and NFOR but all with large drawbacks as there are for example:
**Lactate** is a compound that builds up in the muscle upon workout caused by a shortage of oxygen. The idea is that this building up of lactate in the muscle is a sign of not being able to sufficiently recover. The measurement of lactate only focuses on muscles and not on the whole body response and may well depend on the mode of exercise used and the training status (Urhausen et al., Sports Med.,2002; 32: 95-102). Several companies that develop tests for overreaching make use of whole blood assays, but until the present invention, none of those are able to specifically and reliably measure overreaching, mainly since it is a non-specific effect. Local labs have tried to develop overreaching tests themselves, but there is no notable example of success so far.

**Ratio of glutamine/glutamate.** Glutamin levels may fall with increased training load but it is not a consistent finding. The same applies for the glutamine/glutamate ratio. (Rowbottom et al.; Eur. J. Appl. Physiol, 1995; 70:502-9; Walsh et al.; Sport Med. 1998; 26: 177-91; Smith et al.; Med. Sci. Sports. Exerc., 2000; 32(3): 684-9; Coutts et al.; Int. J. Sports Med.; 2007; 28: 116-24). Commercial glutamine/glutamate testing kits exist as for example offered by Sigma-Aldrich but thorough scientific evidence is lacking (Krzywkowski et al.; Am. J. Physiol. Cell. Physiol, 2001; 281: C1259-C1265).

**Hormones** (including Catecholamines) are considered to be able to measure overreaching but the results are far from consistent. The main reason is that hormones are under influence of many factors such as food intake, gender, age etc. Tests should mainly be invasive which is a huge hurdle for implementing a test like this on the market. Finally, almost all hormones are under feedback control, some by themselves or other hormones or proteins like cytokines. This makes it extremely hard to use hormones as a test to measure one specific cause, like overtraining (Duclos et al.; Int. Sport. Med. J., 2008; 9(2): 56-66).

**Maladaptation of the HPA and other hypothalamic axes and cortisol:** The Hypothalamic-Pituitary-Axis is a neuro-endocrine system and key player in overreaching, localised at the hypothalamic level. One of its function is to cause a stress response, meant to prepare your body for action to flee away from a dangerous (stressful) situation. It's a continuum of disturbance, adaptation and finally maladaptation of these axes that theoretically be an indicator for overreaching (Lehman et al., Med. Sci. Sport Exerc. 1993; 25(7): 854-62. However, the patterns are instable and subject to diurnal rhythm (Douclos et al., J. Appl. Physiol. 2003; 94: 869-75; Duclos et al., Appl. Physiol. Nutr. Metab. 2007; 32: 895-903).

**Cortisol** is a stress hormone that reacts on prolonged levels of high stress, switching the body into the fight or flight modus, inhibiting the immune system in the process. However, cortisol alone has never been shown to be 100% associated with OTS and/or Overreaching. During resting days, cortisol levels in morning plasma and 24 hours urinary free cortisol excretion are normal in endurance trained athletes and are similar to age matched sedentary individuals (Duclos et al., Appl. Physiol. Nutr. Metab. 2007; 32: 895-903).

The currently known methods that aim to measure overreaching have a number of distinct drawbacks. The most important drawback is the lack of convincing evidence that there is a relationship between the biomarker measured and overreaching (lack of specificity). Results are contradictive or only show results under certain circumstances. This is related to the second problem of current methods which is that they are based on only one parameter or one ratio to measure a complex phenotype like overreaching, which makes them extremely vulnerable to the influence of other factors that are unrelated to the effect being monitored. (In a network one parameter is rarely only influenced by one other parameters but is more likely to be influenced by a mix). Following this, the error of the measurements in predicting overreaching can be unacceptably high. Thirdly, this vulnerability to other factors lead to a high variability in outcomes that can be caused by the time of the day, gender and foot patterns. If high vulnerability masks the biological effect the test loses its sensitivity.

There is a strong demand for diagnostic tools to identify athletes in the various training states for FOR, NFOR, and OTS (Meeusen, et al., Med. Sci. Sports Exerc., 2013; 45: 186-205; Soligard et al., Br. J. Sports Med. 2016; 50: 1030-1041). It is hard for athletes to train less if they notice that they are underperforming. Also, for coaches it is hard to distinguish between non-functional overreaching and simple underperformance. The risk is that athletes will simply train more to compensate for lack of performance, which regularly ends up in overtraining with the result that an athlete can't physically train anymore for a period of months or even years.

All measures *used* so far are subjective and are therefore relatively easy to ignore certainly by competitive and driven people. An objective measure that will help the athlete, doctor and/or coach to change the training regimen will therefore greatly add to the performance and health of athletes.

### Summary of the Invention

The invention provides compositions, articles of manufacture, materials, and methods that are useful for improving health of a human subject, and more particularly improving fitness and physical fitness training, and addressing one or more of the unmet or undermet needs described above.

The invention relates to measuring expression of specific proteins in bodily samples. More particular, the invention relates to measuring proteins that are related to the status of the immune system. The invention further relates to means, in particular kits of parts, that enable measuring stress-related proteins in bodily samples.

Also, the use of certain nutritional products that could add to the speed of recovery of athletes is contemplated. A speedy recovery will decrease the chance of non-functional overreaching or worse. Our panel of proteins will be able to identify the effect of such nutritional products or related treatments and will therefore add to the wellbeing and performance of athletes and/or the stressful lives of "driven" people in general.

In one embodiment, the invention also comprises a change of training regimen, like lower work load, longer recovery time or change in exercise mode.

There is a strong demand for diagnostic tools to identify athletes in the various training states for FOR, NFOR, and OTS (Meeusen, et al., Med. Sci. Sports Exerc., 2013; 45: 186-205; Soligard et al., Br. J. Sports Med. 2016; 50: 1030-1041). It is hard for athletes to train less if they notice that they are underperforming. Also, for coaches it is hard to distinguish between non-functional overreaching and simple underperformance. The risk is that athletes will simply train more to compensate for lack of performance, which regularly ends up in overtraining with the result that an athlete can't physically train anymore for a period of months or even years.

All measures *used* so far are subjective and are therefore relatively easy to ignore certainly by competitive and driven people. An objective measure that will help the athlete, doctor and/or coach to change the training regimen will therefore greatly add to the performance and health of athletes.

It has been found that specific proteins that were hitherto unknown to be up-/downregulated as a result of stress can be used in a method for measuring overreaching (see Examples). In particular, the invention provides a method for assigning a sample to a mammal that is suffering from stress, the method comprising: providing a first and at least a second sample comprising a mixture of proteins, mRNA molecules or fragments of proteins or mRNA molecules from a mammal that is about to start a training sequence or is in a training sequence and is at risk of suffering from stress from or during that training sequence.

The invention also provides a kit of parts for determining stress, comprising means for measuring at least two proteins, or corresponding mRNA molecules, fragments of said proteins, or fragments of said mRNA molecules. In particular such kit of parts is suitable for determining protein expression through antibody or aptamer binding.

### Detailed Description of the Invention

A reliable biomarker for FOR, NFOR, and OTS should be sensitive to the training load and occur prior to the establishment of OTS. Additionally, changes in the biomarkers in response to acute exercise should be distinguishable from chronic changes and be relatively easy to collect and measure ((Meeusen, et al.; Med. Sci. Sports Exerc. 2013, 45, 186-205). Up to the present invention, no established objective biomarkers exist for FOR, NFOR, or OTS, and subjective measures are regarded as superior to physiological measures such as plasma hormones and cytokines, energy homeostasis, and exercise workload monitoring (Saw et al., Br. J. Sports Med. 201; 50: 281-291; Joro et al., J. Sports Sci. 2017; 35: 2342-2349; Jürimäe et al., Metabolism. 2011; 60: 335-350; Halson et al., Sports Med. 2004; 34: 967-981).

Thus, there is a need for an alternative method for measuring overreaching and more importantly to use this information to improve the health and performance of athletes. In particular there is a need for an alternative method for measuring overreaching that takes into account the complex biology and heterogeneity of people Not all proteins will react in the same way in all athletes which can depend on for example gender, diet and level of fitness (stratification) among others. There is a need for being able to stratify between athletes to give accurate feedback, which can only be done with more than 1 protein. This results in a stable and highly reliable manner of measuring training load. Our newly proposed overreaching protein panel takes this into account by monitoring *16 different* proteins, that based on our data all have a relation with the intensity of the training load.

The invention provides a method for assigning a sample to a mammal that is suffering from stress, the method comprising: providing a first and at least a second sample comprising a mixture of proteins, mRNA molecules or fragments of proteins or mRNA molecules from a mammal that is about to start a training sequence or is in a training sequence and is at risk of suffering from stress from or during that training sequence. With "sample" is meant a bodily fluid sample that comprises at least the proteins of interest or corresponding mRNA molecule, or a fragment of said protein or mRNA molecule (the fragment being sufficient in size and sequence/structure to identify the protein or mRNA with specificity). A suitable sample is, e.g. blood or blood derived, such as serum or plasma. It is preferred that such bodily fluid sample is easily shipped and analysed. For instance, a dried blood spot (DBS) is a suitable sample. Such dried blood spot is collected without medical intervention by way of a finger prick device on a dedicated receptacle like protein saver cards or Volumetric Absorptive Microsamplers. Around 1 drop of blood, corresponding to ∼10 µl of blood volume, suffices for a measurement. DBS can be kept at ambient temperature for months without the quality of the proteins being affected significantly (Björkesten et al., Mol. Cell Prot. 2017; in press). Proteins can be easily extracted following, for example, the lab protocol described in Example 1.

With "assigning a sample" is meant that, based on the outcome of the protein (fragment) or mRNA (fragment) ratio, a link is made between the sample and the mammal, thereby enabling identifying the mammal as having increased stress (or not), in comparison to a mammal not having such stress. The identification of a mammal as having stress (or not) may be based on the ratio of measurements of a particular protein, corresponding mRNA, or fragments thereof described herein from a second sample with a first sample, but may also be based on measurements from multiple, subsequent samples and one first sample. In another variation, the identification is based on multiple ratios, each being calculated between a particular protein, corresponding mRNA, or fragments thereof measured in a later sample and an earlier sample (i.e., samples taken at different times). For instance, ratios are obtained between a particular protein, corresponding mRNA, or fragments thereof in a second and a first sample, a third and a second sample, a third and a first sample, a fourth and a second sample, and so on. Thus, with "first" and "second" sample is not necessarily meant that it is a very first sample, but it refers to an earlier and a later sample, such as for instance the fifth and tenth sample.

With "training sequence" is meant that a human or another mammal is performing a series of training days, such as for instance during a week training camp. It can also mean a longer series of training days, such as for instance the months before the start of a marathon, or a short series of training days, such as for instance in a weekend training camp. The training sequence is not necessarily limited and can also mean regular yearly training of an athlete or other mammal. Especially for a training sequence with a limited duration, it is preferred that a first sample has been taken before the start of such training sequence and that the last sample has been taken after the end of such training sequence. It is further preferred that several samples are provided that have been taken during the training sequence, in order to act upon discovery of stress during the training sequence. The first sample has preferably been taken after a longer period of rest (preferably more than half a day, more preferably more than 1 day, more preferably more than 2 days, most preferably more than 3 days) and before the first day of training of said training sequence. However, it can also have been taken during the training sequence, preferably in the morning of a day of training or at a day of rest. Said second sample can have been taken after the training sequence, e.g., one or two or more days after the last day of training, or it can have been taken during the training sequence, at a day of training or a day of rest. Preferably it has been taken before the start of a new day of training in the morning before food intake.

The method further comprises: determining the expression ratios of at least two proteins, or corresponding mRNA molecules, fragments of said proteins, or fragments of said mRNA molecules for said second sample or for a sample of said sequence of samples, relative to said first sample. Such determining can easily be accomplished by determining the expression of said at least two proteins, or corresponding mRNA molecules, fragments of said proteins, or fragments of said mRNA molecules in said first and at least second sample and dividing the subsequent sample by said first sample. Determining the expression of a protein or fragment of said protein can, e.g., be performed with spectrometry methods, such as high-performance liquid chromatography (HPLC), liquid chromatography-mass spectrometry (LC/MS), or antibody dependent methods, such as enzyme-linked immunosorbent assay (ELISA), protein immunoprecipitation, immunoelectrophoresis, and protein immunostaining. Determining the expression of a corresponding mRNA molecule or fragment of an mRNA molecule can, e.g., be performed with northern blotting, qPCR or RT-qPCR, hybridization microarray, serial analysis of gene expression (SAGE), next-generation sequencing.

In a method according to the invention, the at least two proteins comprise: Serum amyloid A-4 protein and Inter-alpha-trypsin inhibitor heavy chain H4. The sample under analysis is assigned to a mammal suffering from overreaching if at least one, preferably both, of the expression ratios of said at least two proteins, or corresponding mRNA molecules, fragments of proteins, or fragments of mRNA molecules in said sample under analysis correspond to an expression ratio of at least 1.05, preferably at least 1.1, more preferably at least 1.2. Preferably, the mammal is an equine, a canine or a human subject.

Thus, in a first embodiment, the invention provides a method for assigning a sample to a mammal that is suffering from stress, the method comprising:
a) providing a first sample comprising a mixture of proteins, mRNA molecules or fragments of proteins or mRNA molecules from a mammal that is about to start a training sequence or is in a training sequence, and is at risk of suffering from stress from or during that training sequence,
b) providing a second sample or a sequence of samples comprising a mixture of proteins, mRNA molecules or fragments of proteins or mRNA molecules from said subject during and after the training sequence,
c) determining the expression ratios of at least two proteins, or corresponding mRNA molecules, fragments of said proteins, or fragments of said mRNA molecules for said second sample or for a sample of said sequence of samples, relative to said first sample, wherein the at least two proteins comprise:
   **Serum amyloid A-4 protein and Inter-alpha-trypsin inhibitor heavy chain H4,** and wherein the sample under analysis is assigned to a mammal suffering from stress if at least one, preferably both, of the expression ratios of said at least two proteins, or corresponding mRNA molecules, fragments of proteins, or fragments of mRNA molecules of said sample under analysis correspond to an expression ratio of at least 1.05, preferably at least 1.1, more preferably at least 1.2.

With "determining the expression ratios of at least two proteins" is meant that a ratio is determined of the amount of a first protein in the sample under analysis, relative to that of the same first protein in a sample that had been taken earlier in time; and that a ratio is determined of a second protein in the sample under analysis, relative to that of the same second protein in a sample that had been taken earlier in time. The timepoint of said earlier sample or of the subsequent sample must not necessarily be the same for said first and said second protein.
**Serum amyloid A (SAA) 4:** SAA4 is an acute phase protein. The acute phase response is a systemic reaction to environmental insults including severe stress, infection, trauma, and late-stage cancer, and involves the hepatic production of many proteins including SAA. During the acute phase response, plasma levels of SAA rise to very high levels and trigger multiple signalling pathways related to phagocyte migration and inflammation (Ye et al., J. Leukoc. Biol.; 2015: 98, 923-929). Animal based studies have focused on the value of measuring SAA and other acute phase proteins in monitoring physiological responses to intensified training stress (Cywinska et al., BMC Vet. Res.; 2013: 9, 91; Casella et al., J. Vet. Diagn. Invest.; 2013: 25, 577-580). **ITIH4 (Inter-alpha-trypsin inhibitor heavy chain H4)** is also an acute phase protein. It has, however, been associated with an inflammatory response to trauma, but up to the present invention, not with overtraining or overreaching.

In a preferred embodiment, a method according to the invention is provided, wherein the expression ratio of at least four proteins, or corresponding mRNA molecules, fragments of proteins, or fragments of mRNA molecules is determined for said second sample or for a sample of said sequence of samples, relative to said first sample, wherein the at least four proteins comprise: Serum amyloid A-4 protein, Inter-alpha-trypsin inhibitor heavy chain H4, and two proteins, preferably three proteins, more preferably four proteins selected from the group consisting of: **Alpha-1-acid glycoprotein 2, Complement factor H, Alpha-1-antitrypsin, and Plasma protease C1 inhibitor**, and wherein the sample under analysis is assigned to a mammal suffering from stress if at least three, preferably four, more preferably at least five, most preferably six, of the expression ratios of said at least four proteins, or corresponding mRNA molecules, fragments of proteins, or fragments of mRNA molecules of said sample under analysis correspond to an expression ratio of at least 1.05, preferably at least 1.1, more preferably at least 1.2.

**Alpha-1-acid glycoprotein 2** is a key plasma protein in the acute phase upon inflammation. It may be involved in immune suppression.

**Alpha-1-antitrypsin:** Its primary target is elastase, but it also has a moderate affinity for plasmin and thrombin. Irreversibly inhibits trypsin, chymotrypsin and plasminogen activator. The aberrant form inhibits insulin-induced NO synthesis in platelets, decreases coagulation time and has proteolytic activity against insulin and plasmin.

The complement system is part of the immune system that enhances the ability of the innate immune system to clear microbes etc. and its activation is under control of proteins. We have included **Complement Factor H** to the panel.

Activation of the C1 complex is under control of the C1-inhibitor. This protein plays a potentially crucial role in regulating important physiological pathways including complement activation, blood coagulation, fibrinolysis and the generation of kinins. It's a very efficient inhibitor of the blood clotting factor FXIIa. In our panel we have included the **Plasma protease C1** inhibitor.

In a more preferred embodiment, a method according to the invention is provided, wherein the expression ratio of at least six proteins, or corresponding mRNA molecules, fragments of proteins, or fragments of mRNA molecules is determined for said second sample or for a sample of said sequence of samples, relative to said first sample, wherein the at least six proteins comprise: Serum amyloid A-4 protein, Inter-alpha-trypsin inhibitor heavy chain H4, two proteins, preferably three proteins, more preferably four proteins selected from the group consisting of: Alpha-1-acid glycoprotein 2, Complement factor H, Alpha-1-antitrypsin, Plasma protease C1 inhibitor, and two proteins, preferably three proteins, more preferably four proteins selected from the group consisting of: **Myeloperoxidase, Corticosteroid-binding globulin, Inter-alpha-trypsin inhibitor heavy chain H1, and Alpha-2-HS-glycoprotein**, and wherein the sample under analysis is assigned to a mammal suffering from stress if at least six, preferably seven, more preferably eight, more preferably nine, most preferably ten, of the expression ratios of said at least six proteins, or corresponding mRNA molecules, fragments of proteins, or fragments of mRNA molecules of said sample under analysis correspond to an expression ratio of at least 1.05, preferably at least 1.1, more preferably at least 1.2.

**Myeloperoxidase (MPO):** Myeloperoxidase is central to the microbicidal activity of neutrophils.

In our panel we have included **Corticosteroid-binding globulin** which is an important transport protein of glucocorticoids. Glucocorticoids act as modulators for inflammatory events resulting from the combined action of the autonomic nervous system, and the hypothalamic-pituitary-adrenal axis. The immune system itself, through the action of cytokines and other factors, in turn, activates the CNS to orchestrate negative-feedback mechanisms that keep the immune response in check. Disruption of these interactions has been associated with a number of syndromes including inflammatory, autoimmune, and cardiovascular diseases, metabolic and psychiatric disorders, and the development of shock (Tait et al., J. Leukoc. Biol., 2008; 84(4): 924-931).
In this group we also include two proteins involved in the acute phase; **Inter-alpha-trypsin inhibitor heavy chain H1** has been implicated in multiple inflammatory diseases. **Alpha-2-HS-glycoprotein** promotes endocytosis and is also part of phagocytosis.

In a most preferred embodiment, a method according to the invention is provided, wherein the expression ratio of at least eight proteins, or corresponding mRNA molecules, fragments of proteins, or fragments of mRNA molecules is determined for said second sample or for a sample of said sequence of samples, relative to said first sample, wherein the at least eight proteins comprise: Serum amyloid A-4 protein, Inter-alpha-trypsin inhibitor heavy chain H4, two proteins, preferably three proteins, more preferably four proteins selected from the group consisting of: Alpha-1-acid glycoprotein 2, Complement factor H, Alpha-1-antitrypsin, Plasma protease C1 inhibitor, two proteins, preferably three proteins, more preferably four proteins selected from the group consisting of: Myeloperoxidase, Corticosteroid-binding globulin, Inter-alpha-trypsin inhibitor heavy chain H1, Alpha-2-HS-glycoprotein, and two proteins, preferably three proteins, more preferably four proteins, more preferably five proteins, most preferably six proteins selected from the group consisting of: **Alpha-1-Antichymotrypsin, Fibronectin, Fibrinogen gamma chain, Complement component C8 gamma chain, Complement C4B, and Complement component C7** and wherein the sample under analysis is assigned to a mammal suffering from stress if at least eight, preferably ten, more preferably twelve, more preferably thirteen, more preferably fourteen, more preferably at least fifteen, most preferably sixteen, of the expression ratios of said at least eight proteins, or corresponding mRNA molecules, fragments of proteins, or fragments of mRNA molecules of said sample under analysis correspond to an expression ratio of at least 1.05, preferably at least 1.1, more preferably at least 1.2.

**Alpha-1-Antichymotrypsin.** This protein can inhibit neutrophil cathepsin G and mast cell chymase, both of which can convert angiotensin-1 to the active angiotensin-2.

**Fibrinogen gamma chain** and **Fibronectin** are both involved in wound healing. Fibronectins bind cell surfaces and various compounds including collagen, fibrin, heparin, DNA, and actin. Fibronectins are involved in cell adhesion, cell motility, opsonization, wound healing, and maintenance of cell shape. Fibrinogen gamma chain polymerizes to form an insoluble fibrin matrix. It has a major function in hemostasis as one of the primary components of blood clots. In addition, it functions during the early stages of wound repair to stabilize the lesion and guide cell migration during re-epithelialization.

We have found several complement component proteins namely **Complement component C8 gamma chain, Complement C4B, Complement component C7.** The complement system is part of the immune system that enhances the effect of antibodies and phagocytotic cells to clean microbes and damaged cells and to promote inflammation.

As described above, there are many methods available to determine the expression of proteins, or corresponding protein fragments, mRNA molecules, or fragments of mRNA molecules. Minor differences in sensitivity and specificity between such methods may lead to slightly different expression ratios. The ratios claimed are determined by means of mass spectrometry. Therefore, in a preferred embodiment, a method according to the invention is provided, wherein said ratio is at least 1.05, preferably at least 1.1, more preferably at least 1.2, when determined with Mass Spectrometry (MS) using the following protocol: solubilizing proteins from a dried blood spot in a denaturing buffer pH=8.0, comprising the mixture of proteins, mRNA molecules or fragments of proteins or mRNA molecules. Proteins will subsequently be digested into peptides (see Example 1) and measured with Liquid Chromatograpy (LC)-MS/MS (see example 2). During the liquid chromatography step, peptides are separated over a ReversePhase column based on their physico-chemical properties to simplify the peptide mixture. Because of this step the peptides (representing the proteins) are analysed by the mass spectrometer over the course of 10 minutes to 4 hours. The peptides can be measured using different ion mode being Parallel Reaction Monitoring, Multiple Reaction Monitoring and Selective Reaction monitoring based on the type of MS instrument used. These are targeted methods only targeting the peptides representing the proteins in our panel only. Alternatively, the peptides can be measured by Data Dependent Acquisition, a discovery based approach that detects all peptides in the sample technically possible including the peptides representing the proteins in this panel. Also, the peptides can be measured by Data Independent Acquisition, a discovery based approach less limited by the complexity of the sample than DDA that detects all peptides in the sample as is technically possible including the peptides representing the proteins in this sample. The protein quantitation is defined by calculating the areas underneath the peak representing the peptides of interest and monitoring the change of the quantitative values of the proteins over time. The protein quantitation is then compared between timepoints to discover an up or down going trend in response to the training load. The method according to the invention is suitable for determining stress in a mammal, preferably a horse, a dog or a human subject, more preferably a human subject. Stress in this context can be any of the following: 1) Physiological disturbance caused to an organism by adverse circumstances, like for example demanding exercise or change in exercise volume; 2) A state of emotional or mental strain/tension caused by adverse and/or demanding circumstances, like a demanding work place or negative external emotional influences. In a preferred embodiment, therefore, a method according to the invention is provided, wherein stress is selected from the group consisting of physical stress, caused by, e.g., sports, training, or (physical) trauma, and psychological stress caused by, e.g., work or psychological trauma. In a particularly preferred embodiment, the physical stress is selected from the group of functional overreaching, non-functional overreaching and overtraining.
Functional OverReaching (FOR) is defined as overreaching coupled to adequate recovery time leading to higher levels of fitness and greater resistance to illness and stress: Repeated moderate exercise has a positive impact on the immune system in the long run, via periodic stimulation and adaptation. Non-Functional Overreaching (NFOR) has exactly the opposite effect - the lack of an adequate recovery time causes training to have an adverse effect on performance and increases vulnerability to illness and stress. If the situation of NFOR is not taken into consideration by the athlete/coach/trainer and/or medical doctor it ends up in overtraining or "the OverTraining Syndrome (OTS)" with prolonged (weeks to months) or even permanent absence from sports and/or work. The invention can be used for a wide array or sports which includes endurance sports like running, cycling, swimming and rowing. It can also be used for other sports that are physical demanding like weightlifting and triathlons. It can also be used for races with animals as the central point of focus like horse races and dog races. Often these animals are subjected to heavy training regimen without the possibility to have a subjective measurement.
In all these cases the test could lead to the conclusion that the training is too tough which could lead to a change in training regimen. Examples of a change in training regimen are, for instance, inserting longer periods of rest between sessions or during the training week, decreasing the training load by a certain percentage that will make the athlete recover quicker or inserting a different kind of exercise that breaks the monotonicity of the training scheme, for example by inserting short swimming sessions or weight sessions for a cycling athlete. Also new training schedules can be developed that take into account these stress levels that they cause based on the described panel. The coach/athlete can use this panel to see how this change of training scheme affects recovery and training status objectively. Since the panel as described here consists of several proteins it has the possibility to personalise and stratify treatments (like nutrition) and changes in training schedule based on the individual measurements, which thus far does not exist.

Now that the invention provides a method for determining stress, the invention further provides a kit of parts for determining stress. In one embodiment, the invention provides a kit of parts, comprising means for measuring at least two proteins, or corresponding mRNA molecules, fragments of said proteins, or fragments of said mRNA molecules, wherein the at least two proteins comprise: Serum amyloid A-4 protein and Inter-alpha-trypsin inhibitor heavy chain H4. In particular such kit of parts is suitable for determining protein expression through antibody or aptamer binding. Exemplary "means for measuring" proteins or corresponding fragments of said proteins include antibodies or aptamers that specifically bind the protein (i.e. they do not appreciably cross-react with other proteins that may be present in the sample from the subject. The term "antibody" includes, without limitation, an intact immunoglobulin molecule such as a polyclonal or monoclonal antibody, an antigen-binding functional fragment, including, but not limited to, Fab, F(ab'), F(ab')2 , Fv, dAb, Fd, a complementarity determining region (CDR) fragment, a single chain antibody (scFv), a divalent single chain antibody, a single chain phage antibody, a bispecific double chain antibody, a triabody, a tetrabody, a single domain antibody (nanobody®), a (poly)peptide containing at least an amino acid sequence that is sufficient to specifically bind to its target, and artificial immunoglobulin fragments, such as plastic antibodies [Padlan et al (1991) Methods Enzymol 203:3-21]. An "aptamer" is a RNA- of DNA-molecule that specifically binds to a ligand, such as the proteins or protein fragments of interest to this invention. A strip or a protein array able to measure proteins via one of the methods as mentioned above like antibodies and such. Such kit of parts may also be suitable to determine protein expression by determining the amount of a corresponding mRNA or mRNA fragment. For determining the amount of mRNA (fragments), a primer pair and/or probe may be used and quantitative polymerase chain reaction (qPCR) or reverse transcriptase (RT)-qPCR may be employed. Primers/probes are selected for specificity for the target mRNA or mRNAs, based on the known gene/mRNA sequence and preferably also based on knowledge of the species' genome (to avoid false positives). Thus, according to a preferred embodiment, a kit of parts according to the invention is provided, wherein said means for measuring at least two proteins, or corresponding mRNA molecules, fragments of said proteins, or fragments of said mRNA molecules, is a binding molecule, preferably selected from the group consisting of an antibody, a primer pair, a probe, or an aptamer.

As explained above, determination of other proteins may be combined in order to increase the specificity and sensitivity of a method according to the invention. The same holds true for the kit of parts. Therefore, in a preferred embodiment, a kit of parts according to the invention is provided, comprising means for measuring at least four proteins, or corresponding mRNA molecules, fragments of said proteins, or fragments of said mRNA molecules, wherein the at least four proteins comprise: Serum amyloid A-4 protein, Inter-alpha-trypsin inhibitor heavy chain H4, and two proteins, preferably three proteins, more preferably four proteins selected from the group consisting of: Alpha-1-acid glycoprotein 2, Complement factor H, Alpha-1-antitrypsin, and Plasma protease C1 inhibitor.

In a more preferred embodiment, a kit of parts according to the invention is provided, comprising means for measuring at least six proteins, or corresponding mRNA molecules, fragments of said proteins, or fragments of said mRNA molecules, wherein the at least six proteins comprise: Serum amyloid A-4 protein, Inter-alpha-trypsin inhibitor heavy chain H4, two proteins, preferably three proteins, more preferably four proteins selected from the group consisting of: Alpha-1-acid glycoprotein 2, Complement factor H, Alpha-1-antitrypsin, Plasma protease C1 inhibitor, and two proteins, preferably three proteins, more preferably four proteins selected from the group consisting of: Myeloperoxidase, Corticosteroid-binding globulin, Inter-alpha-trypsin inhibitor heavy chain H1, and Alpha-2-HS-glycoprotein.

In a most preferred embodiment, a kit of parts according to the invention is provided, comprising means for measuring at least eight proteins, or corresponding mRNA molecules, fragments of said proteins, or fragments of said mRNA molecules, wherein the at least eight proteins comprise: Serum amyloid A-4 protein, Inter-alpha-trypsin inhibitor heavy chain H4, two proteins, preferably three proteins, more preferably four proteins selected from the group consisting of: Alpha-1-acid glycoprotein 2, Complement factor H, Alpha-1-antitrypsin, Plasma protease C1 inhibitor, two proteins, preferably three proteins, more preferably four proteins selected from the group consisting of: Myeloperoxidase, Corticosteroid-binding globulin, Inter-alpha-trypsin inhibitor heavy chain H1, Alpha-2-HS-glycoprotein, and two proteins, preferably three proteins, more preferably four proteins, more preferably five proteins, most preferably six proteins selected from the group consisting of: Alpha-1-Antichymotrypsin, Fibronectin, Fibrinogen gamma chain, Complement component C8 gamma chain, Complement C4B, and Complement component C7.

Such kits are especially useful for determining stress in a mammal. Subsequently, athletes, coaches or other persons can act in order to attenuate the stress and increase performance in said mammal. Such acting is described in more detail above.

In one embodiment, the invention provides a method of measuring a state of physical stress in a mammalian subject, the method comprising:
comparing first measurements of at least two biomarkers obtained from a first biological sample from the mammalian subject with second measurements of said at least two biomarkers obtained from a second biological sample from the mammalian subject,
wherein the first biological sample has been obtained prior to at least one session of physical exertion,
wherein the second biological sample has been obtained subsequent to the at least one session of physical exertion,
wherein one of the at least two biomarkers comprises Serum amyloid A-4 protein or Serum amyloid A-4 mRNA; and
wherein one of the at least two biomarkers comprises Inter-alpha-trypsin inhibitor; heavy chain H4 protein, or Inter-alpha-trypsin inhibitor heavy chain H4 mRNA; and
diagnosing the state of physical stress as functional overreaching (FOR), nonfunctional overreaching (NFOR) or overtraining syndrome (OTS) from the comparison of the measurements of the two or more biomarkers.

Preferably, said comparing comprises determining a ratio of the measurement of each biomarker in the second sample relative to the measurement in the first sample. Preferably, the method comprises diagnosing FOR, NFOR, or OTS from a ratio of at least 1.05, or at least 1.1, or at least 1.2 for at least one of said at least two biomarkers, preferably for two of the at least two biomarkers.

In one embodiment, the method comprises comparing first measurements of at least four biomarkers obtained from the first biological sample with second measurements of said at least four biomarkers obtained from the second biological sample,
wherein the at least four biomarkers comprise two, three, or four proteins selected from the group consisting of Alpha-1-acid glycoprotein 2, Complement factor H, Alpha-1-antitrypsin, and Plasma protease C1; or comprise mRNA corresponding to said proteins. Preferably, said comparing comprises determining, for each biomarker, a ratio of the measurement in the second sample relative to the measurement in the first sample. Preferably the method comprises diagnosing FOR, NFOR, or OTS from a ratio of at least 1.05, or at least 1.1, or at least 1.2 for at least three, at least four, at least five, or at least six of said biomarkers.

In one embodiment, the method comprises comparing first measurements of at least six biomarkers obtained from the first biological sample with second measurements of said at least six biomarkers obtained from the second biological sample,
wherein the at least six biomarkers comprise two, three, or four proteins selected from the group consisting of Myeloperoxidase, Corticosteroid-binding globulin, Inter-alpha-trypsin inhibitor heavy chain H1, and Alpha-2-HS-glycoprotein; or comprise mRNA corresponding to said proteins. Preferably, the comparing comprises determining, for each biomarker, a ratio of the measurement in the second sample relative to the measurement in the first sample. Preferably, the method comprises diagnosing FOR, NFOR, or OTS from a ratio of at least 1.05, or at least 1.1, or at least 1.2 for at least 6, 7, 8, 9, or 10 of said biomarkers.

In one embodiment, the method comprises comparing first measurements of at least eight biomarkers obtained from the first biological sample with second measurements of said at least eight biomarkers obtained from the second biological sample,
wherein the at least eight biomarkers comprise 2, 3, 4, 5, or 6 proteins selected from the group consisting of Alpha-1-Antichymotrypsin, Fibronectin, Fibrinogen gamma chain, Complement component C8 gamma chain, Complement C4B, and Complement component C7; or comprise mRNA corresponding to said proteins. Preferably, the comparing comprises determining, for each biomarker, a ratio of the measurement in the second sample relative to the measurement in the first sample. Preferably, the method comprises diagnosing FOR, NFOR, or OTS from a ratio of at least 1.05, or at least 1.1, or at least 1.2 for at least 8, 9, 10, 11, 12, 13, 14, 15, or 16 of said biomarkers.

In one embodiment, a method according to the invention is provided, wherein the first and second biological samples each comprises blood, serum, or plasma, preferably dried blood, more preferably less than 75 µl, less than 50 µl, less than 40 µl, less than 30 µl, less than 25 µl, or less than 20 µl of dried blood.

In one embodiment, a method according to the invention is provided, further comprising obtaining the second biological sample and measuring the two or more biomarkers in the second biological sample.

In one embodiment, a method according to the invention is provided, further comprising obtaining the first biological sample and measuring the two or more biomarkers in the first biological sample.

In one embodiment, a method according to the invention is provided, wherein the two or more biomarkers are proteins and wherein, preferably the measuring of a protein biomarker comprises measuring at least one peptide fragment of the protein biomarker via mass spectrometry (MS). Preferably, the biological sample comprises dried blood, and the MS comprises: solubilizing proteins from the dried blood, in a denaturing buffer; fragmenting the proteins into peptides; and measuring the peptides with Liquid Chromatograpy(LC)-MS/MS, wherein preferably the fragmenting comprises digesting the proteins with a protease and, preferably, the protein quantitation is defined by calculating the areas underneath the peak representing the peptides of interest. Preferably, the measuring of the peptide comprises comparing the measurement of the peptide to a measurement of standard, wherein the standard comprises a known quantity of said peptide that is isotopically labeled (see Example 3).

In one embodiment, the peptides are measured by immunoassay.

In one embodiment, a method according to the invention is provided, wherein the second biological sample is obtained from the subject between one and five days, preferably between one and four days, more preferably between one and three days, more preferably between one and two day, most preferably one day after completing the physical exertion. In one embodiment, the physical exertion comprises cardiovascular training or weight training. In one embodiment, the physical exertion comprises at least one exercise selected from weight lifting, running, and cycling.

In one embodiment, the invention provides a method of physical fitness training for a mammalian subject comprised of multiple episodes of physical exertion separated by periods of rest, the improvement that comprises:
measuring physical stress one or more times according to a method according to the invention;
for a subject diagnosed with NFOR, or OTS, reducing the intensity or the frequency of the episodes of physical exertion, or increasing the length of the periods of rest.

In one embodiment, the invention provides a method of physical fitness training for a mammalian subject comprised of multiple episodes of physical exertion separated by periods of rest, the improvement that comprises:
measuring physical stress one or more times according to a method according to the invention;
for a subject diagnosed with FOR monitoring the training of the episodes of physical exertion, and pushing the exertions to the limit.

In one embodiment, the invention provides a method of physical fitness training for a mammalian subject comprised of multiple episodes of physical exertion separated by periods of rest, the improvement that comprises:
measuring physical stress one or more times according to a method according to the invention;
for a subject not diagnosed with FOR, NFOR, or OTS - increasing the intensity or frequency of the episodes of physical exertion, or decreasing the periods of rest.

In one embodiment, the invention provides an individualized physical fitness program for a plurality of subjects, the program comprising:
exercising a plurality of exercise episodes;
resting between exercise episodes for rest periods;
measuring physical stress one or more times according to a method according to the invention, for each subject;
for at least one subject diagnosed with NFOR, or OTS, reducing the intensity or the frequency of the exercise episodes, or increasing the length of the periods of rest; and
for at least one subject diagnosed with FOR, monitoring the training of the episodes of physical exertion, and pushing the exertions to the limit, and
for at least one subject not diagnosed with FOR, NFOR, or OTS increasing the intensity or frequency of the episodes of physical exertion, or decreasing the periods of rest.

In one embodiment, the invention provides for an isolated peptide comprising an amino acid sequence set forth in Table 1, with the proviso that the peptide comprises an isotopic label that changes the molecular weight of the peptide, compared to the predominant molecular weight of a corresponding peptide isolatable from fragmentation of a wildtype protein (see Example 3).

In a preferred embodiment, the isolated peptide according to the invention is provided, wherein the isotopic label comprises ¹³C, ¹⁵N.

In one embodiment, the invention provides a kit comprising at least 2, 3, 4, 5, 6, 7, 8,9, 10, 11, 12, 13, 14, 15, or 16 peptides according to the invention.

In a preferred embodiment, a kit according to the inventions is provided, further comprising peptide resolubilisation buffer.

### Figure Legends

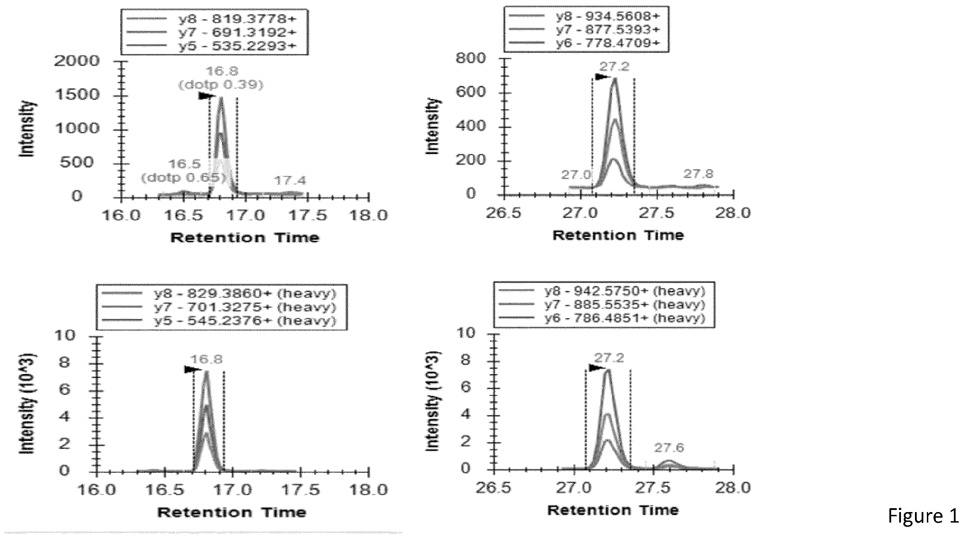

Fig. 1: Upper panels show the identification of a peptide representing SAA4 on the left and ITIH4 on the right. The lower panels show the heavy labelled spiked-in peptides representing the same peptide as the upper panel. The heavy labelled peptide is used for identification, normalisation and quality control. In SAA4 the most intense signal in the graphic represents the fragment ions y6, then y5 and the lowest intense signal is y8. For ITIH4 the most intense fragment ion is y6, then y8 and the lowest intense signal is y7.

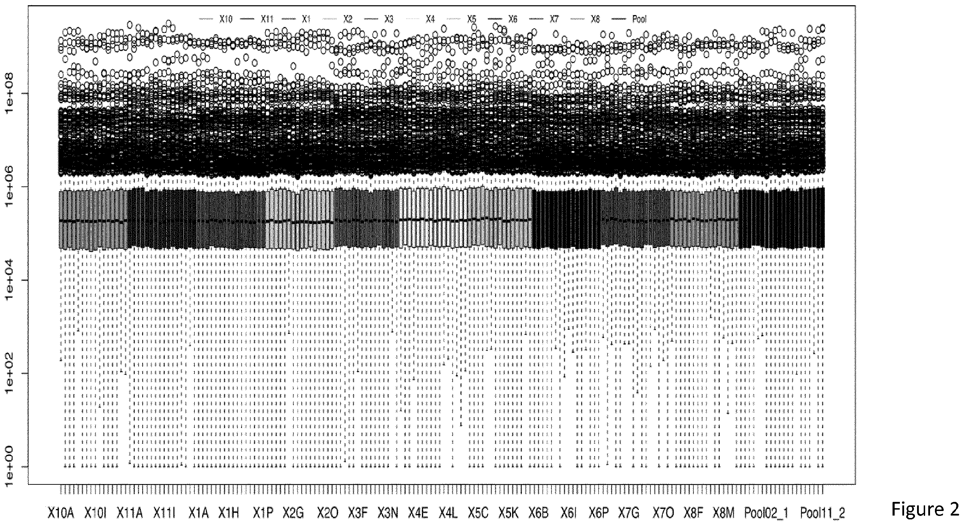

Fig. 2: The use of pools for batch correction for DIA experiments results in low technical variability. Box plots of the intensities of all proteins measured (y-axis) throughout all samples (x-axis) show a very constant signal.

### TABLES

**Table 1 - List of proteins and peptides that are useful in a method according to the invention**

| **Group** | **UniProt Protein** | **Protein Name** | **SEQ ID NO:** | **Sequence** | **SEQ ID NO:** | **m/z** | **z** |
|---|---|---|---|---|---|---|---|
| **1** | P35542 | Serum amyloid A-4 protein | 1 | EALQGVGDM GR | 81 | 566.77 | 2 |
| | | | | GPGGVWAAK | 82 | 421.73 | 2 |
| **1** | Q14624 | Inter-alpha-trypsin inhibitor heavy chain H4 | 2 | LGVYELLLK | 83 | 524.33 | 2 |
| | | | | SPEQQETVLD GNLIIR | 84 | 906.48 | 2 |
| | | | | ILDDLSPR | 85 | 464.76 | 2 |
| **2** | P19652 | Alpha-1-acid glycoprotein 2 | 3 | WFYIASAFR | 86 | 580.8 | 2 |
| | | | | EHVAHLLFLR | 87 | 412.24 | 2 |
| **2** | P08603 | Complement factor H | 4 | IDVHLVPDR | 88 | 532.3 | 2 |
| | | | | SSNLIIILEEHL K | 89 | 698.4 | 2 |
| | | | | EIMENYNIAL R | 90 | 683.35 | 2 |
| **2** | P01009 | Alpha-1-antitrypsin | 5 | DTEEEDFHVD QVTTVK | 91 | 631.29 | 3 |
| | | | | SVLGQLGITK | 92 | 508.31 | 2 |
| | | | | VFSNGADLSG VTEEAPLK | 93 | 917.47 | 2 |
| **2** | P05155 | Plasma protease C1 inhibitor | 6 | TNLESILSYPK | 94 | 632.84 | 2 |
| | | | | LLDSLPSDTR | 95 | 558.8 | 2 |
| **3** | P05164 | Myeloperoxi dase | 7 | VVLEGGIDPIL R | 96 | 640.88 | 2 |
| **3** | P08185 | Corticosteroi d-binding globulin | 8 | HLVALSPK | 97 | 432.77 | 2 |
| | | | | SETEIHQGFQ HLHQLFAK | 98 | 717.36 | 3 |
| | | | | WSAGLTSSQV DLYIPK | 99 | 882.96 | 2 |
| **3** | P19827 | Inter-alpha-trypsin inhibitor heavy chain H1 | 9 | FAHYVVTSQV VNTANEAR | 100 | 669.34 | 3 |
| | | | | AAISGENAGL VR | 101 | 579.32 | 2 |
| | | | | GFSLDEATNL NGGLLR | 102 | 838.93 | 2 |
| **3** | P02765 | Alpha-2-HS-glycoprotein | 10 | FSVVYAK | 103 | 407.23 | 2 |
| **4** | P01011 | Alpha-1-Antichymotr ypsin | 11 | LYGSEAFATD FQDSAAAK | 104 | 946.44 | 2 |
| | | | | EIGELYLPK | 105 | 531.3 | 2 |
| | | | | ADLSGITGAR | 106 | 480.76 | 2 |
| **4** | P02751 | Fibronectin | 12 | NLQPASEYTV SLVAIK | 107 | 578.32 | 3 |
| | | | | SYTITGLQPG TDYK | 108 | 772.39 | 2 |
| | | | | FLATTPNSLL VSWQPPR | 109 | 964.03 | 2 |
| 4 | P02679 | Fibrinogen gamma chain | 13 | YEASILTHDSS IR | 110 | 497.92 | 3 |
| | | | | IHLISTQSAIP YALR | 111 | 841.98 | 2 |
| **4** | P07360 | Complement component C8 gamma chain | 14 | SLPVSDSVLS GFEQR | 112 | 810.92 | 2 |
| | | | | VQEAHLTEDQ IFYFPK | 113 | 655.66 | 3 |
| **4** | P0C0L5 | Complement C4B | 15 | QGSFQGGFR | 114 | 492.24 | 2 |
| | | | | GLQDEDGYR | 115 | 526.74 | 2 |
| **4** | P10643 | Complement component C7 | 16 | SYTSHTNEIH K | 116 | 658.82 | 2 |
| | | | | VLFYVDSEK | 117 | 550.29 | 2 |
| | | | | AASGTQNNVL R | 118 | 565.8 | 2 |
| | | | | YSAWAESVTN LPQVIK | 119 | 903.48 | |

**Table 2 - List of corresponding equine proteins that are useful in a method according to the invention (numbers refer to SEQ ID NOs).**

| | |
|---|---|
| 17 | SAA_HORSE Serum amyloid A protein |
| 18 | F6R3F4_HORSE Inter-alpha-trypsin inhibitor heavy chain family member 4 |
| 19 | A1AT1_HORSE Alpha-1-antiproteinase 1 (Fragments) |
| 20 | F7BZ88_HORSE Myeloperoxidase |
| 21 | 6QX36_HORSE Inter-alpha-trypsin inhibitor heavy chain 1 |
| 22 | F7C450_HORSE Alpha 2-HS glycoprotein |
| 23 | FINC_HORSE Fibronectin (Fragment) |
| 24 | F6W2Y1_HORSE Fibrinogen gamma chain |
| 25 | F7D2W0_HORSE Complement C3b/C4b receptor 1 (Knops blood group) |
| 26 | F6Q7J3_HORSE Complement C7 |

**Table 3 - List of corresponding canine proteins that are useful in a method according to the invention (numbers refer to SEQ ID NOs).**

| | |
|---|---|
| 27 | SAA_CANLF Serum amyloid A protein |
| 28 | H9GWY1_CANLF Inter-alpha-trypsin inhibitor heavy chain family member 4 |
| 29 | A1ILJ0_CANLF Alpha 1 antitrypsin |
| 30 | F1PQ52_CANLF Myeloperoxidase |
| 31 | F1Q418_CANLF Inter-alpha-trypsin inhibitor heavy chain 1 |
| 32 | E2QUV3_CANLF Alpha 2-HS glycoprotein |
| 33 | FINC_CANLF Fibronectin (Fragment) |
| 34 | F1P8G0_CANLF Fibrinogen gamma chain |
| 35 | E2R0W5_CANLF CO4B |
| 36 | E2RG-1_CANLF Complement C7 |

**Table 4 - List of genes that encode for the proteins that are useful in a method according to the invention (numbers refer to SEQ ID NOs). The list of human genes are in the same order as the corresponding proteins in Table 1.**

| Human | Equine | Canine |
|---|---|---|
| 37 SAA4 | 51 SAA4 | 66 SAA4 |
| 38 ITIH4 | 52 ITIH4 | 67 ITIH4 |
| 39 A1AG2 | 53 CFAH | 68 CFAH |
| 40 CFAH | 54 A1AT | 69 A1AT |
| 41 A1AT | 55 IC1 | 70 IC1 |
| 42 IC1 | 56 Myeloperoxidase | 71 Myeloperoxidase |
| 43 Myeloperoxidase | 57 CBG | 72 CBG |
| 44 CBG | 58 ITIH1 | 73 ITIH1 |
| 45 ITIH1 | 59 FETUA | 74 FETUA |
| 46 AHSG (FETUA) | 60 AACT | 75 AACT |
| 120 AACT | 61 Fibronectin | 76 Fibronectin |
| 121 Fibronectin | 62 Fibrinogen gamma chain | 77 Fibrinogen gamma chain |
| 47 Fibrinogen gamma chain | 63 CO8G | 78 CO8G |
| 48 CO8G | 64 CO4B | 79 CO4B |
| 49 CO4B | 65 CO7 | 80 CO7 |
| 50 CO7 | | |

### EXAMPLES

### Example 1:

**Proteomics Procedures:** Proteins are solubilized from DBS in 8M Urea, 50mM ammonium bicarbonate buffer, pH=8.0, reduced and alkylated with 10mM DTT and IodoAcetamide respectively, and digested into peptides using trypsin (∼1 molecule of trypsin per -50 molecules of protein). Samples are cleaned up using C18 columns and after elution in 50% Acentonitrile (ACN)/ 0.1% Formic Acid (FA) peptides are dried down by speedvac. Before injection into the MS sample are resolubilised in 3%ACN and 0.1% FA. To reduce variance, all samples are randomized and subsequently measured consecutively by mass spectrometry (MS). This results in a clean tryptic protein digest to the high standards as demanded by MS.

### Example 2:

**LC-MS:** 1µg of peptides are separated on a nano-LC column over a 60 minutes gradient starting with 2% ACN and ending with 40% ACN. Peptides are measured in MS1 at 70000 resolution, AGC target of 1e6 and 250ms trapping time. Peptides are measured in MS2 at 17500 resolution, AGC target of 5e4 and 120 ms trapping time.

Data are acquired either in Data Independent Acquisition mode (DIA) or Parallel Reaction Monitoring (PRM). Data Independent Acquisition means that the data acquired is not dependent on a topN most intense precursor peptides but instead acquires all peptide information available. Heavy isotopically labelled standards are used for Parallel Reaction Monitoring (PRM). PRM means that the peptide of interest is selected only for data acquisition with a pre-determined isolation window. MS analyses are performed on a Q-Exactive mass spectrometer (Thermo Fisher Scientific, Waltham, MA) coupled to a nanoLC autosampler (Dionex Corporation, Sunnyvale, CA). 1µg of DBS peptide of each sample is injected and peptides are separated with reverse phase nanoLC chromatography. All samples are measured with data independent acquisition mode (DIA) or with parallel reaction monitoring (PRM).

For PRM, only the peptides of interest are measured with an isolation window of 1.2 Th. In DIA the isolation windows are 25 Th with a total number of 29 windows (400-1150). The result in DIA is the identification and quantitation of at least 400 proteins including the protein panel as presented in this patent. In PRM mode the protein panel as presented here is identified and quantified.

### Example 3:

**QC for Targeted MS (PRM/ SRM/MRM):** Heavy isotopically labelled peptides are spiked in and used for identification, quantitation and normalisation. Heavy labelled peptide are specifically synthesized for use of the method and/or the kit. Heavy isotopically labelled peptides are here defined as peptides with exactly the same sequence as the endogenous naturally occurring sequence with the modification of a C-terminal Arginine (R) or Lysine (K) of which all nitrogen (¹⁴N) and carbon (¹²C) atoms are replaced by their heavier isotope (¹⁵N; ¹³C) which makes the total mass 10 and 8 Th heavier respectively in order to be able to distinguish in a protein sample between endogenous peptides and the spiked in standards.

The reasons for using the heavy labelled peptides in Parallel Reaction Monitoring are three fold:
1) Quality control of the performance of the MS to measure drift of the signal over time.
2) Normalisation of the endogenous signal to the constant heavy standard peptide signal.
3) Precise high confident identification of peptides (and hence proteins)

**Example 4: QC for Data Independent Acquisition:** MS sensitivity and precision are monitored using a pooled sample of DBS samples, with injections after twelve runs when data are required in a Data Independent Acquisition mode. This results in quantitative data with the lowest variation technically possible. Pools can be used for batch correction and normalisation. This results in reliable data to the highest standards with a low technical variability below the biological effect.

**Example 5: Data processing:** The DIA files are processed using SpectronautTM software (Biognosys). Spectronaut is also used to calculate the false discovery rate (FDR) of identified peptides and a cut-off of 0.01. For each protein, the three most abundant peptides are used for quantitative analysis (in case more than three peptides per protein were identified). For DIA a human spectral library is required to process the data. They are either custom made using Spectronaut or are downloaded on-line as pre-existing databases. For PRM, data is analysed in Skyline software.

**Example 6: Measurement of athletes that have suffered from OTS:** Samples are serum based and/or are as under Example 1-4. Results give well defined protein signatures for OTS. These experiments include the measurement of serum and DBS of athletes running at high pace for a week, running at normal pace for a week and resting for a week. Differences in proteins signature between resting, normal exercise and extreme exercise are measured. These experiments also include the measurement of serum and/or DBS to measure the influence of nutrition on the recovery rate of intense training athletes. Nutrition can increase the recovery rate which is measured by use of our proteins panel.

### Results:

**Table 5: Effect of intense training on protein expression.** Day 1 till day 3 are training intense days consisting of 2,5 hours of exercise. Blood samples were measured before (morning) and after (afternoon) exercise. On day 4 and day 5 there was no exercise and a blood sample was taken only in the morning. Athletes were their own control, meaning they either trained or "sat in a chair" (control) and the other way around. Blood samples were always taken at the same time in control and training. The proteins in our panel respond not or little in the first three training days whereas they increase in expression on the resting days after exercise, an effect that does not occur in the control.

Data are ratios of median intensity of the protein signal on the day divided by the median intensity of all measurements.

| | | **Training** | **Training** | **Training** | **Training** | **Training** | **Training** | **Training** | **Training** |
|---|---|---|---|---|---|---|---|---|---|
| **Protein ID** | **Protein name** | **Day1 Mor** | **Day1 Aft** | **Day2 Mor** | **Day2 Aft** | **Day3 Mor** | **Day3 Aft** | **Day4 Mor** | **Day5 Mor** |
| P35542 | SAA4 | 0.95 | 0.86 | 0.93 | 1.01 | 1.00 | 1.03 | 1.16 | 1.17 |
| Q14624 | ITIH4 | 0.99 | 0.96 | 0.94 | 1.01 | 1.04 | 1.01 | 1.05 | 1.10 |
| P19652 | A1AG2 | 0.97 | 0.89 | 0.99 | 0.99 | 1.00 | 1.05 | 1.07 | 1.07 |
| P01009 | A1AT | 1.00 | 0.94 | 0.95 | 0.97 | 1.00 | 1.06 | 1.06 | 1.10 |
| P05155 | IC1 | 1.00 | 0.99 | 0.99 | 0.98 | 1.02 | 1.00 | 1.10 | 1.10 |
| P05164 | PERM | 0.89 | 1.05 | 0.99 | 1.14 | 0.99 | 1.06 | 1.01 | 1.42 |
| P08185 | CBG | 0.97 | 0.94 | 1.00 | 1.01 | 1.00 | 1.03 | 1.11 | 1.17 |
| P19827 | ITIH1 | 1.02 | 0.97 | 0.95 | 0.93 | 0.98 | 0.96 | 1.04 | 1.01 |
| P02765 | FETUA | 0.97 | 0.99 | 0.96 | 1.02 | 1.01 | 1.07 | 1.09 | 1.05 |
| P01011 | AACT | 0.91 | 0.98 | 0.94 | 0.99 | 1.01 | 1.09 | 1.01 | 1.04 |
| P02751 | FINC | 0.97 | 0.99 | 1.01 | 0.98 | 1.01 | 0.98 | 1.01 | 1.06 |
| P07360 | CO8 | 0.99 | 0.92 | 0.94 | 0.97 | 1.04 | 1.03 | 1.11 | 1.13 |
| P0C0L5 | CO4B | 0.97 | 0.86 | 0.98 | 0.97 | 1.04 | 0.99 | 1.11 | 1.07 |
| P10643 | CO7 | 1.00 | 0.85 | 0.97 | 0.99 | 0.98 | 0.94 | 1.09 | 1.09 |

| | | **Control** | **Control** | **Contol** | **Control** | **Control** | **Control** | **Control** | **Control** |
|---|---|---|---|---|---|---|---|---|---|
| **Protein ID** | **Protein name** | **Day1 Mor** | **Day1 Aft** | **Day2 Mor** | **Day2 Aft** | **Day3 Mor** | **Day3 Aft** | **Day4 Mor** | **Day5 Mor** |
| P35542 | SAA4 | 1.00 | 0.99 | 0.97 | 0.98 | 0.98 | 1.01 | 0.97 | 1.01 |
| Q14624 | ITIH4 | 0.98 | 0.95 | 0.99 | 0.96 | 0.99 | 1.05 | 1.00 | 1.00 |
| P19652 | A1AG2 | 0.98 | 0.93 | 0.94 | 1.01 | 1.02 | 1.00 | 0.98 | 1.01 |
| P01009 | A1AT | 0.98 | 1.00 | 0.93 | 0.95 | 1.02 | 1.02 | 1.01 | 1.02 |
| P05155 | IC1 | 1.01 | 1.05 | 0.93 | 0.98 | 1.07 | 0.93 | 1.00 | 1.00 |
| P05164 | PERM | 1.13 | 1.24 | 1.14 | 0.96 | 0.96 | 0.86 | 0.92 | 1.06 |
| P08185 | CBG | 1.00 | 0.97 | 1.00 | 0.91 | 1.04 | 0.96 | 1.03 | 1.02 |
| P19827 | ITIH1 | 0.99 | 0.99 | 0.96 | 1.02 | 1.05 | 1.04 | 1.04 | 1.01 |
| P02765 | FETUA | 0.95 | 0.95 | 0.90 | 0.95 | 1.03 | 0.95 | 0.98 | 1.03 |
| P01011 | AACT | 0.95 | 1.00 | 0.96 | 1.02 | 0.96 | 1.00 | 1.04 | 1.02 |
| P02751 | FINC | 1.00 | 0.98 | 1.02 | 1.00 | 1.01 | 0.98 | 1.00 | 1.01 |
| P07360 | CO8 | 0.98 | 0.98 | 0.96 | 0.93 | 1.00 | 1.03 | 1.00 | 1.03 |
| P0C0L5 | CO4B | 0.96 | 1.04 | 0.99 | 1.02 | 1.00 | 0.98 | 1.00 | 1.02 |
| P10643 | CO7 | 1.01 | 0.94 | 1.00 | 0.97 | 0.93 | 1.01 | 0.99 | 1.08 |

**Table 6**: **Effect of intense training on the expression of two proteins of a longitudinal over several weeks.** Swimmers trained over a time course of a couple of weeks and each Monday after the rest day a blood sample was taken. Training intensity changed upon the schedule as created by the coach. Both proteins show a clear upregulation when training intensity was high.

Data are ratios of median intensity of the protein signal on the day divided by the median intensity of all measurements.

| **Protein ID** | **Protein name** | **Median/all time median** | **Training Intensity (Coach)** |
|---|---|---|---|
| P08603 | CFAH | 0.71 | low |
| P08603 | CFAH | 0.72 | low |
| P08603 | CFAH | 0.80 | low |
| P08603 | CFAH | 0.87 | low |
| P08603 | CFAH | 0.94 | low |
| P08603 | CFAH | 0.99 | low |
| P08603 | CFAH | 1.00 | low |
| P08603 | CFAH | 1.04 | low |
| P08603 | CFAH | 1.05 | low |
| P08603 | CFAH | 1.06 | high |
| P08603 | CFAH | 1.08 | high |
| P08603 | CFAH | 1.10 | high |
| P08603 | CFAH | 1.11 | high |
| P08603 | CFAH | 1.27 | high |
| P08603 | CFAH | 1.27 | high |
| P02679 | FIBG | 0.32 | low |
| P02679 | FIBG | 0.51 | low |
| P02679 | FIBG | 0.52 | low |
| P02679 | FIBG | 0.53 | low |
| P02679 | FIBG | 0.66 | low |
| P02679 | FIBG | 0.71 | low |
| P02679 | FIBG | 0.75 | low |
| P02679 | FIBG | 0.87 | low |
| P02679 | FIBG | 1.00 | low |
| P02679 | FIBG | 1.14 | high |
| P02679 | FIBG | 1.17 | high |
| P02679 | FIBG | 1.21 | high |
| P02679 | FIBG | 1.37 | high |
| P02679 | FIBG | 1.42 | high |
| P02679 | FIBG | 1.68 | high |

## Claims

1. Method for assigning a sample to a mammal that is suffering from stress, the method comprising:
a) providing a first sample comprising a mixture of proteins, mRNA molecules or fragments of proteins or mRNA molecules from a mammal that is about to start a training sequence or is in a training sequence, and is at risk of suffering from stress from or during that training sequence,
b) providing a second sample or a sequence of samples comprising a mixture of proteins, mRNA molecules or fragments of proteins or mRNA molecules from said subject during and after the training sequence,
c) determining the expression ratios of at least two proteins, or corresponding mRNA molecules, fragments of said proteins, or fragments of said mRNA molecules for said second sample or for a sample of said sequence of samples, relative to said first sample, wherein
the at least two proteins comprise: Serum amyloid A-4 protein and Inter-alpha-trypsin inhibitor heavy chain H4, and wherein the sample under analysis is assigned to a mammal suffering from overreaching if at least one, preferably both, of the expression ratios of said at least two proteins, or corresponding mRNA molecules, fragments of proteins, or fragments of mRNA molecules of said sample under analysis correspond to an expression ratio of at least 1.05, preferably at least 1.1, more preferably at least 1.2.

2. A method according to claim 1, wherein the expression ratio of at least four proteins, or corresponding mRNA molecules, fragments of proteins, or fragments of mRNA molecules is determined for said second sample or for a sample of said sequence of samples, relative to said first sample, wherein the at least four proteins comprise: Serum amyloid A-4 protein, Inter-alpha-trypsin inhibitor heavy chain H4, and two proteins, preferably three proteins, more preferably four proteins selected from the group consisting of: Alpha-1-acid glycoprotein 2, Complement factor H, Alpha-1-antitrypsin, and Plasma protease C1 inhibitor, and wherein the sample under analysis is assigned to a mammal suffering from stress if at least three, preferably four, more preferably at least five, most preferably six, of the expression ratios of said at least four proteins, or corresponding mRNA molecules, fragments of proteins, or fragments of mRNA molecules of said sample under analysis correspond to an expression ratio of at least 1.05, preferably at least 1.1, more preferably at least 1.2.

3. Method according to claim 1, wherein the expression ratio of at least four proteins, or corresponding mRNA molecules, fragments of proteins, or fragments of mRNA molecules is determined for said second sample or for a sample of said sequence of samples, relative to said first sample, wherein the at least six proteins comprise: Serum amyloid A-4 protein, Inter-alpha-trypsin inhibitor heavy chain H4, two proteins, preferably three proteins, more preferably four proteins selected from the group consisting of: Alpha-1-acid glycoprotein 2, Complement factor H, Alpha-1-antitrypsin, Plasma protease C1 inhibitor, and two proteins, preferably three proteins, more preferably four proteins selected from the group consisting of: Myeloperoxidase, Corticosteroid-binding globulin, Inter-alpha-trypsin inhibitor heavy chain H1, and Alpha-2-HS-glycoprotein, and wherein the sample under analysis is assigned to a mammal suffering from stress if at least six, preferably seven, more preferably eight, more preferably nine, most preferably ten, of the expression ratios of said at least six proteins, or corresponding mRNA molecules, fragments of proteins, or fragments of mRNA molecules of said sample under analysis correspond to an expression ratio of at least 1.05, preferably at least 1.1, more preferably at least 1.2.

4. Method according to claim 1, wherein the expression ratio of at least four proteins, or corresponding mRNA molecules, fragments of proteins, or fragments of mRNA molecules is determined for said second sample or for a sample of said sequence of samples, relative to said first sample, wherein the at least eight proteins comprise: Serum amyloid A-4 protein, Inter-alpha-trypsin inhibitor heavy chain H4, two proteins, preferably three proteins, more preferably four proteins selected from the group consisting of: Alpha-1-acid glycoprotein 2, Complement factor H, Alpha-1-antitrypsin, Plasma protease C1 inhibitor, two proteins, preferably three proteins, more preferably four proteins selected from the group consisting of: Myeloperoxidase, Corticosteroid-binding globulin, Inter-alpha-trypsin inhibitor heavy chain H1, Alpha-2-HS-glycoprotein, and two proteins, preferably three proteins, more preferably four proteins, more preferably five proteins, most preferably six proteins selected from the group consisting of: Alpha-1-Antichymotrypsin, Fibronectin, Fibrinogen gamma chain, Complement component C8 gamma chain, Complement C4B, and Complement component C7 and wherein the sample under analysis is assigned to a mammal suffering from stress if at least eight, preferably ten, more preferably twelve, more preferably thirteen, more preferably fourteen, more preferably at least fifteen, most preferably sixteen, of the expression ratios of said at least eight proteins, or corresponding mRNA molecules, fragments of proteins, or fragments of mRNA molecules of said sample under analysis correspond to an expression ratio of at least 1.05, preferably at least 1.1, more preferably at least 1.2.

5. Method according to any of claim 1 - 4, wherein said ratio is at least 1.05, preferably at least 1.1, more preferably at least 1.2, when determined with Mass Spectrometry (MS) using the following protocol: solubilizing proteins from a dried blood spot comprising the mixture of proteins, mRNA molecules or fragments of proteins or mRNA molecules, in a denaturing buffer, pH=8, digesting the proteins into peptides and measure the peptides with Liquid Chromatograpy(LC)-MS/MS, wherein the protein quantitation is defined by calculating the areas underneath the peak representing the peptides of interest and monitoring the change of the quantitative values of the proteins over time.

6. Method according to any one of claims 1 - 5, wherein stress is selected from the group consisting of physical stress, caused by, e.g., sports, training, or (physical) trauma, and psychological stress caused by, e.g., work or psychological trauma.

7. Method according to claim 6, wherein the physical stress is selected from the group of functional overreaching, non-functional overreaching, and overtraining.

8. Method according to any one of claim 1 - 6, wherein the expression ratio of not more than 1000, preferably not more than 500, more preferably not more than 200, more preferably not more than 100, more preferably not more than 50, most preferably not more than 20 proteins, or corresponding mRNA molecules, fragments of proteins, or fragments of mRNA molecules is determined.

9. Kit of parts, comprising means for measuring at least two proteins, or corresponding mRNA molecules, fragments of said proteins, or fragments of said mRNA molecules,
wherein
the at least two proteins comprise: Serum amyloid A-4 protein and Inter-alpha-trypsin inhibitor heavy chain H4.

10. Kit of parts according to claim 9, comprising means for measuring at least four proteins, or corresponding mRNA molecules, fragments of said proteins, or fragments of said mRNA molecules, wherein the at least four proteins comprise: Serum amyloid A-4 protein, Inter-alpha-trypsin inhibitor heavy chain H4, and two proteins, preferably three proteins, more preferably four proteins selected from the group consisting of: Alpha-1-acid glycoprotein 2, Complement factor H, Alpha-1-antitrypsin, and Plasma protease C1 inhibitor.

11. Kit of parts according to claim 10, comprising means for measuring at least six proteins, or corresponding mRNA molecules, fragments of said proteins, or fragments of said mRNA molecules, wherein the at least six proteins comprise: Serum amyloid A-4 protein, Inter-alpha-trypsin inhibitor heavy chain H4, two proteins, preferably three proteins, more preferably four proteins selected from the group consisting of: Alpha-1-acid glycoprotein 2, Complement factor H, Alpha-1-antitrypsin, Plasma protease C1 inhibitor, and two proteins, preferably three proteins, more preferably four proteins selected from the group consisting of: Myeloperoxidase, Corticosteroid-binding globulin, Inter-alpha-trypsin inhibitor heavy chain H1, and Alpha-2-HS-glycoprotein.

12. Kit of parts according to claim 11, comprising means for measuring at least eight proteins, or corresponding mRNA molecules, fragments of said proteins, or fragments of said mRNA molecules, wherein the at least eight proteins comprise: Serum amyloid A-4 protein, Inter-alpha-trypsin inhibitor heavy chain H4, two proteins, preferably three proteins, more preferably four proteins selected from the group consisting of: Alpha-1-acid glycoprotein 2, Complement factor H, Alpha-1-antitrypsin, Plasma protease C1 inhibitor, two proteins, preferably three proteins, more preferably four proteins selected from the group consisting of: Myeloperoxidase, Corticosteroid-binding globulin, Inter-alpha-trypsin inhibitor heavy chain H1, Alpha-2-HS-glycoprotein, and two proteins, preferably three proteins, more preferably four proteins, more preferably five proteins, most preferably six proteins selected from the group consisting of: Alpha-1-Antichymotrypsin, Fibronectin, Fibrinogen gamma chain, Complement component C8 gamma chain, Complement C4B, and Complement component C7.

13. Kit of parts according to any one of claims 9 - 12, wherein said means for measuring at least two proteins, or corresponding mRNA molecules, fragments of said proteins, or fragments of said mRNA molecules, is a binding molecule, preferably selected from the group consisting of an antibody, a primer pair, a probe, or an aptamer.

14. Kit of parts according to any one of claim 9 - 13, comprising means for measuring at most 1000, preferably at most 500, more preferably at most 200, more preferably at most 100, more preferably at most 50, most preferably at most 20 proteins, or corresponding mRNA molecules, fragments of said proteins, or fragments of said mRNA molecules.
